# EUROPÄISCHE PATENTANMELDUNG

(11) **EP 4 234 528 A1**
(43) Veröffentlichungstag der Anmeldung: **30.08.2023**
(21) Anmeldenummer: 23154876.9
(22) Anmeldetag: 03.02.2023
(51) Int. Cl.: C07C 31/125, C07C 29/141

(54) **VERFAHREN ZUR HYDRIERUNG VON C13-ALDEHYDEN IN MINDESTENS ZWEI HYDRIERSTUFEN**

(30) Priorität: 25.02.2022 EP 22158736
(71) Anmelder: Evonik Operations GmbH, 45128 Essen (DE)
(72) Erfinder: Franke, Robert, 45772 Marl (DE); Roos, Meike, 63654 Büdingen (DE); Zanthoff, Horst-Werner, 45481 Mülheim a.d. Ruhr (DE); Bauer, Julia, 45721 Haltern am See (DE); Weber, Christoph, 65183 Wiesbaden (DE); Heinroth, Andrea, 63776 Möbris (DE); Metternich, Jan Benedikt, 45657 Recklinghausen (DE)
(74) Vertreter: Evonik Patent Association

(57) **Zusammenfassung**

Die vorliegende Erfindung betrifft ein Verfahren zur Herstellung von Alkoholen durch Hydrierung von C13-Aldehyden. Das erfindungsgemäße Verfahren wird in zwei aufeinanderfolgenden Hydrierstufen durchgeführt, wobei in der ersten Hydrierstufe ein aktivierter Metallkatalysator auf Basis eines Nickelmetallschaums und in der zweiten Stufe ein Trägerkatalysator, der eine katalytisch aktive Komponente aus der Gruppe bestehend aus Nickel, Kupfer, Chrom und Mischungen davon enthält, eingesetzt wird.

## Beschreibung

Gegenstand der Erfindung ist ein Verfahren zur Herstellung von Alkoholen durch Hydrierung von C13-Aldehyden. Das erfindungsgemäße Verfahren wird in zwei aufeinanderfolgenden Hydrierstufen durchgeführt, wobei in der ersten Hydrierstufe ein aktivierter Metallkatalysator auf Basis eines Nickelmetallschaums und in der zweiten Stufe ein Trägerkatalysator, der eine katalytisch aktive Komponente aus der Gruppe bestehend aus Nickel, Kupfer, Chrom und Mischungen davon enthält, eingesetzt wird.

Die grundlegende Aufgabe der Petrochemie ist die Bereitstellung von Grundstoffen für die chemische Industrie. Dazu gehört die Herstellung von Aldehyden, die beispielsweise mittels Hydroformylierung von Olefinen erhalten werden. Die so erhaltenen Aldehyde können dann mittels Hydrierung zu Alkoholen weiterverarbeitet werden. Industriell wird die Hydrierung von Aldehyden üblicherweise an im Festbett angeordneten heterogenen Katalysatoren in der Gas- oder Flüssigphase durchgeführt. Dabei kommt sowohl dem Katalysator als auch der Fahrweise in den damit betriebenen Reaktionsapparaten eine entscheidende Bedeutung für den Prozess zu. Die Katalysatoren bestimmen beispielsweise die intrinsische Reaktionsgeschwindigkeit und Selektivität der Hydrierung. Zudem ist die Auswahl eines geeigneten Katalysators auch deshalb wichtig, da die zu hydrierenden Aldehyde meistens als ein Gemisch von strukturisomeren Aldehyden und potenziell störenden Nebenprodukten, die zum einen in der Hydrierung unerwünschte Nebenreaktionen hervorrufen und zum anderen den Hydrierkatalysator schädigen können, eingesetzt werden. Die Fahrweise der eingesetzten Reaktionsapparate ermöglicht beispielsweise Einfluss auf Konzentrationen, Stoff- und Wärmetransportvorgänge im Reaktionssystem zu nehmen und dadurch die intrinsischen Eigenschaften der Katalysatoren optimal zu nutzen.

Für die Hydrierung von Aldehyden sind dem Fachmann bereits eine Vielzahl von verschiedenen Katalysatoren bekannt, beispielsweise Mischmetallkatalysatoren wie in der EP 3 037 400 A1 oder der WO 2011/045102 A1 oder aktivierte Metallkatalysatoren wie in der WO 2007/028411 A1. Üblicherweise werden unter aktivierten Metallkatalysatoren Metalllegierungen verstanden, die auf metallische, oxidische oder kohlenstoffhaltige Träger aufgebracht worden sind und durch Laugung aktiviert werden, wobei der Träger vollständig entfernt werden kann.

Als eingesetzte Reaktionsapparate sind je nach gewollter Einflussnahme auf die Reaktion und die parallel ablaufenden Stoff- und Wärmetransportprozesse eine Vielzahl von Reaktortypen und Kombinationen von Reaktortypen möglich, wie z.B. in DE102004059292A1 beschrieben.

Es besteht ein kontinuierlicher Bedarf an Verfahrensverbesserungen bei der Hydrierung von Aldehyden. Das Hydrierverfahren sollte sich durch eine gute Aktivität und Alkohol-Selektivität auszeichnen und dadurch, dass möglichst wenig unerwünschte Nebenprodukte gebildet werden und/oder möglichst viele unerwünschte Nebenprodukte während des Verfahrens abgebaut werden.

Diese Aufgabe wird durch das erfindungsgemäße Verfahren nach Anspruch 1 gelöst. Bevorzugte Ausführungsformen sind in den Unteransprüchen angegeben. Das erfindungsgemäße Verfahren ist ein Verfahren zur Herstellung von Alkoholen durch kontinuierliche Hydrierung von C13-Aldehyden in mindestens zwei Hydrierstufen, wobei

ein Strom, der die zu hydrierenden C13-Aldehyde enthält, in flüssiger Phase in der ersten Hydrierstufe, die mindestens einen Kreislaufreaktor umfasst, mit einem Wasserstoff-haltigen Gas an einem aktivierten Metallkatalysator auf Basis eines Nickelmetallschaums hydriert wird, wobei aus dem mindestens einen Kreislaufreaktor ein Rohproduktstrom, zumindest enthaltend Alkohole und nicht umgesetzte Aldehyde, entnommen wird, von dem ein erster Teil zurückgeführt und ein zweiter Teil zur zweiten Hydrierstufe geführt wird,
der zweite Teil des Rohproduktstroms in flüssiger Phase in der zweiten Hydrierstufe, die mindestens einen im geraden Durchgang betriebenen Reaktor umfasst, mit einem Wasserstoff-haltigen Gas an einem Trägerkatalysator hydriert wird, der eine katalytisch aktive Komponente und ein Trägermaterial umfasst, wobei die katalytisch aktive Komponente aus der Gruppe bestehend aus Nickel, Kupfer, Chrom und Mischungen davon ausgewählt wird, und wobei das Trägermaterial zu mehr als 90 Gew.-% aus einem oxidischen Material besteht, welches aus der Gruppe bestehend aus Aluminiumoxid, Aluminiumsilikat, Siliciumdioxid, Titandioxid, Zirkonoxid und Mischungen von zwei oder mehr davon, ausgewählt wird.

Für das Verfahren wird ein Strom eingesetzt, der die zu hydrierenden C13-Aldehyde enthält und zur ersten Hydrierstufe geführt wird. Derartige Ströme können z. B. einer vorgeschalteten kontinuierlichen oder diskontinuierlichen Hydroformylierung von C12-Olefinen entstammen. Nach der Hydroformylierung kann zumindest eine Abtrennung des dabei üblicherweise eingesetzten homogenen Katalysators erfolgen. Bei Einsatz von heterogenisierten Katalysatorsystemen, wie sie beispielsweise in der EP 3 632 885 A1 offenbart werden, ist eine Abtrennung des Katalysatorsystems hingegen nicht erforderlich. Verfahren zur Herstellung der erfindungsgemäß eingesetzten Aldehyde durch Hydroformylierung sind dem Fachmann geläufig und werden hier nicht genauer beschrieben. In einer bevorzugten Ausführungsform der vorliegenden Erfindung ist der Strom, der die zu hydrierenden C13-Aldehyde enthält, ein Isotridecanal-Strom. Ein Isotridacanal-Strom im Sinne der vorliegenden Erfindung ist eine Mischung aus verschiedenen isomeren C13-Aldehyden. Solche Mischungen werden großtechnisch durch Hydroformylierung aus C12-Olefinfraktionen erhalten, wobei die C12-Olefine beispielsweise über eine Oligomerisierung von C4-Olefinen, also Butenen, zugänglich sind. Diese Verfahren sind dem Fachmann geläufig.

Der eingesetzte Aldehyd-haltige Strom wird in der ersten Hydrierstufe unter Verwendung eines aktivierten Metallkatalysators auf Basis eines Nickelmetallschaums hydriert. Solche Katalysatoren sind beispielsweise aus der EP 2 764 916 A1 bekannt, die dort als oberflächenmodifizierte Metallschaumkörper bezeichnet werden. Die Herstellung dieser Katalysatoren kann beispielsweise durch das nachfolgend beschriebene Verfahren erfolgen:
a) Ein kommerziell erhältlicher Nickelmetallschaum wird mit einem Haftvermittler (z. B. Polyvinylpyrrolidon oder ein Polyethylenimin) behandelt und anschließend mit Aluminiumpulver beschichtet, wobei das Aufbringen des Aluminiumpulvers bevorzugt durch Sprühen, Bestreuen oder Gießen erfolgt und wobei das Aluminiumpulver zu 90 bis 99,8 Gew.-% aus Aluminiumpartikeln besteht und einen Sauerstoffgehalt von 0,01 bis 0,85 Gew.-% aufweist, jeweils bezogen auf das Gesamtgewicht des Aluminiumpulvers. Besonders bevorzugt weisen die im Aluminiumpulver enthaltenen Aluminiumpartikel eine Partikelgröße im Bereich von 5 µm bis 200 µm auf mit einem d₉₀-Wert im Bereich von 50 bis 75 µm.
b) In einer anschließenden Wärmebehandlung unter Sauerstoffausschluss wird Aluminium im Nickelmetallschaum unter Ausbildung intermetallischer Phasen gelöst und zugleich der Haftvermittler entfernt. Struktur und Porenstruktur des Nickelmetallschaums bleiben dabei vollständig erhalten. Die Wärmebehandlung kann bei einer Temperatur im Bereich von 500 bis 1000 °C durchgeführt werden und erfolgt bevorzugt in mehreren Stufen bei unterschiedlichen Temperaturen, wobei in einer ersten Stufe der Haftvermittler entfernt wird ("Entbinderung"), und in einer darauffolgenden Stufe mit höherer Temperatur das Lösen des Aluminiums im Nickelschaum unter Ausbildung intermetallischer Phasen erfolgt. Besonders bevorzugt wird eine Maximaltemperatur von 800°C während der gesamten Wärmebehandlung nicht überschritten
c) Danach kann eine Zerkleinerung und/oder Vereinzelung des Materials erfolgen, sofern dies nicht bereits in einem Formgebungsschritt vor der Wärmebehandlung geschehen ist. Zur Zerkleinerung des Materials können thermische oder mechanische Schneidverfahren verwendet werden. Bevorzugt erfolgt die Zerkleinerung durch Laserschneiden beziehungsweise Laserstrahlschneiden oder mit Hilfe geeigneter Schneidmesser.
d) Der eigentliche Katalysator wird im letzten Schritt durch Herauslösen mindestens eines Teils des in der Legierung enthaltenen Aluminiums erzeugt. Hierzu werden wässrige basische Lösungen eingesetzt, bevorzugt Alkalihydroxidlösungen, beispielsweise hergestellt durch Lösen von Natriumhydroxid, Kaliumhydroxid oder Lithiumhydroxid in Wasser. Die Konzentration der in diesem Prozessschritt eingesetzten wässrigen Alkalihydroxidlösungen liegt allgemein in einem Bereich zwischen 0,1 bis 60 Gew. -%. Bevorzugt erfolgt das Herauslösen des Aluminiums mit einer 5 bis 50 Gew.-%igen, besonders bevorzugt 5 bis 25 Gew.-%igen wässrigen Natriumhydroxidlösung bei einer Temperatur im Bereich von 20 bis 100 °C, bevorzugt in einem Bereich von 40 bis 85 °C, besonders bevorzugt in einem Bereich von 50 bis 70 °C. Die Reaktionszeiten der Natriumhydroxidlösung mit dem mit Aluminium legierten Nickelmetallschaum können zwischen 5 und 300 Minuten liegen. Bevorzugt liegt die Reaktionszeit der Natriumhydroxidlösung mit dem mit Aluminium legierten Nickelschaum im Bereich von 30 und 180 Minuten.

In einer bevorzugten Ausführungsform der vorliegenden Erfindung ist der in der ersten Hydrierstufe eingesetzte aktivierte Metallkatalysator auf Basis eines Nickelmetallschaums frei von organischen Bestandteilen, d. h. die Summe der Gewichtsanteile von Kohlenstoff und kohlenstoffhaltigen Verbindungen beträgt weniger als 0,2 Gew.-% des Gesamtgewichts des Katalysators. Diese Eigenschaft bezieht sich auf den Katalysator direkt nach der Herstellung und damit vor dem Einsatz in der Hydrierung. Während der Hydrierung kann sich der aktivierte Metallkatalysator auf Basis eines Nickelmetallschaums mit organischen Bestandteilen belegen, also höhere Anteile an kohlenstoffhaltigen Verbindungen aufweisen.

Der aktivierte Metallkatalysator auf Basis eines Nickelmetallschaums gemäß der vorliegenden Erfindung enthält vorzugsweise 80 bis 95 Gew.-% Nickel, 5 bis 15 Gew.-% Aluminium und optional 0 bis 5 Gew.-% Promotoren, wie z. B. Kupfer oder Molybdän, jeweils bezogen auf das Gesamtgewicht des Katalysators. In einer bevorzugten Ausführungsform enthält der aktivierte Metallkatalysator auf Basis eines Nickelmetallschaums zusätzlich 0,01 bis 3 Gew.-% Molybdän, besonders bevorzugt 0,2 bis 1,5 Gew.-% Molybdän und ganz besonders bevorzugt 0,3 bis 0,7 Gew.-% Molybdän, jeweils bezogen auf das Gesamtgewicht des Katalysators.

Strukturell ist der in der ersten Hydrierstufe eingesetzte aktivierte Metallkatalysator auf Basis eines Nickelmetallschaums grundsätzlich keinen Beschränkungen unterworfen, solange ein ausreichender Kontakt mit den umzusetzenden Aldehyden gewährleistet ist. Es ist jedoch bevorzugt, wenn der aktivierte Metallkatalysator auf Basis eines Nickelmetallschaums eine BET-Oberfläche von 1 bis 200 m²/g, bevorzugt 5 bis 100 m²/g, besonders bevorzugt 15 bis 80 m²/g aufweist. Die BET-Oberfläche kann bekanntermaßen mittels Gasadsorption bestimmt werden. Für den aktivierten Metallkatalysator auf Basis eines Nickelmetallschaums kann es aufgrund seiner Eigenschaften notwendig sein, ihn unter Wasser zu lagern.

In dem erfindungsgemäßen Hydrierverfahren werden unterschiedliche Katalysatoren in den beiden Hydrierstufen eingesetzt. Dabei ist es vorteilhaft, wenn der Volumenanteil des aktivierten Metallkatalysators auf Basis eines Nickelmetallschaums am Gesamtkatalysatorvolumen aller Hydrierstufen von 30 bis 80%, vorzugsweise 35 bis 60% beträgt. Dadurch lässt sich eine besonders effiziente Reaktionsführung erreichen.

Die Hydrierung in der ersten Hydrierstufe wird in mindestens einem Kreislaufreaktor durchgeführt, bei dem ein Teil des Rohproduktstroms zurückgeführt wird, eingesetzt. In einer bevorzugten Ausführungsform der vorliegenden Erfindung besteht die erste Hydrierstufe aus einem Kreislaufreaktor. Der aktivierte Metallkatalysator auf Basis eines Nickelmetallschaums wird in dem mindestens einen Kreislaufreaktor als feste Schüttung oder als strukturierte Packung eingesetzt.

Die Hydrierung in der ersten Hydrierstufe kann im Allgemeinen bei einem Druck von 5 bis 150 bar, vorzugsweise 15 bis 50 bar, besonders bevorzugt 20 bis 45 bar durchgeführt werden. Ganz besonders bevorzugt ist ein Druck von 20 bis 30 bar. Die Temperatur bei der Hydrierung in der ersten Hydrierstufe des erfindungsgemäßen Verfahrens liegt vorzugsweise im Bereich von 50 bis 250 °C, vorzugsweise 80 bis 200 °C, besonders bevorzugt von 100 bis 190 °C.

Die Hydrierung in der ersten Hydrierstufe kann weiterhin in Gegenwart eines unter den Hydrierbedingungen inerten Lösungsmittels durchgeführt werden. Inerte Lösungsmittel sind dem Fachmann bekannt, werden aber vorzugsweise aus der Gruppe, bestehend aus Kohlenwasserstoffen und Alkoholen, vorzugsweise den aus den eingesetzten Aldehyden erhaltenen Alkoholen ausgewählt. Beim Einsatz zumindest eines Kreislaufreaktors kann der aus der Hydrierung erhaltene Alkohol auch als Lösungsmittel fungieren. Darüber hinaus kann die Hydrierung in Anwesenheit einer wässrigen Phase, beispielsweise Prozesswasser aus der vorherigen Herstellung der Aldehyde oder der Hydrierung selbst, durchgeführt werden. Es ist erfindungsgemäß aber bevorzugt, dass zur Hydrierung in der ersten Stufe keine zusätzliche wässrige Phase hinzugefügt wird.

Das für die Hydrierung in der ersten Hydrierstufe eingesetzte Wasserstoff-haltige Gas kann sowohl Wasserstoff sein als auch ein Gasgemisch, welches neben Wasserstoff ein oder mehrere unter den Hydrierbedingungen inerte Gase enthält. Klar sollte sein, dass die Menge an Wasserstoff hoch genug ist, um die Hydrierung in ausreichendem Maße durchführen zu können. Es ist weiterhin bevorzugt, wenn der Wasserstoff im Hinblick auf die zu hydrierenden Aldehyde in einem gewissen stöchiometrischen Überschuss eingesetzt wird. Der stöchiometrische Überschuss an Wasserstoff im Vergleich zu den zu hydrierenden Aldehyden liegt vorzugsweise im Bereich von 5 bis 90%, besonders bevorzugt zwischen 20 und 70%.

Unter den angegebenen Verfahrensbedingungen in der ersten Hydrierstufe lassen sich hohe Reaktionsumsätze erzielen. Vorzugsweise beträgt der Umsatz in der Hydrierung in der ersten Hydrierstufe mindestens 85%, vorzugsweise mindestens 90%, besonders bevorzugt mindestens 95%.

Aus der ersten Hydrierstufe wird ein Rohproduktstrom entnommen, der zumindest Alkohole und nicht umgesetzte Aldehyde enthält. Zumindest ein Teil dieses Stroms wird zur zweiten Hydrierstufe geführt und dort einer zweiten Hydrierung unterworfen. Da in der ersten Hydrierstufe ein Kreislaufreaktor vorhanden ist, wird ein erster Teil des Rohproduktstroms zurückgeführt und ein zweiter Teil des Rohproduktstroms zur zweiten Hydrierstufe geführt.

In der zweiten Hydrierstufe des erfindungsgemäßen Verfahrens wird mindestens ein im geraden Durchgang betriebener Reaktor eingesetzt, um zumindest einen Teil der noch nicht hydrierten Aldehyde umzusetzen. Zudem können in der zweiten Stufe Nebenprodukte aus der Hydroformylierung oder der ersten Hydrierstufe umgesetzt und dadurch abgebaut werden. Für die vorliegende Erfindung ist das zum Beispiel die Acetalspaltung, mit der als Nebenprodukt vorliegende Acetale entfernt werden. Dazu wird ein geeigneter Hydrierkatalysator eingesetzt, der sich vom Katalysator in der ersten Hydrierstufe unterscheidet und der eine katalytisch aktive Komponente und ein Trägermaterial umfasst.

Die katalytische aktive Komponente wird erfindungsgemäß aus der Gruppe bestehend aus Nickel, Kupfer, Chrom und Mischungen davon ausgewählt. In einer bevorzugten Ausführungsform der vorliegenden Erfindung ist der Katalysator frei von Chrom, d. h. enthält, bezogen auf die Gesamtzusammensetzung aus zumindest katalytisch aktiver Komponente und Trägermaterial, weniger als 50 Gew.-ppm Chrom. Besonders bevorzugt ist die katalytisch aktive Komponente eine Mischung aus Kupfer und Nickel. Entsprechende Mischungen von Nickel und Kupfer enthaltende Katalysatoren sind beispielsweise in der EP 3 037 400 A1 offenbart, wo auch die Herstellung eines derartigen Katalysators beschrieben wird.

Das Trägermaterial des in der zweiten Hydrierstufe eingesetzten Trägerkatalysators besteht zu mehr als 90 Gew.-% aus einem oxidischen Material, welches aus der Gruppe, bestehend aus Aluminiumoxid, Aluminiumsilikat, Siliciumdioxid, Titandioxid, Zirkonoxid und Mischungen von zwei oder mehr davon, ausgewählt wird. Das Trägermaterial für den Trägerkatalysator ist vorzugsweise Aluminiumoxid, Aluminiumsilikat oder Siliciumdioxid. In einer besonders bevorzugten Ausführungsform wird als Trägermaterial Aluminiumoxid eingesetzt. Das für den Katalysator eingesetzte Trägermaterial kann dabei eine BET-Oberfläche von 70 bis 350 m²/g, vorzugsweise 150 bis 280 m²/g aufweisen. Die Bestimmung der BET-Oberfläche kann bekanntermaßen mittels Gasadsorption gemessen werden.

Der in der zweiten Hydrierstufe eingesetzte Trägerkatalysator kann weitere Stoffe enthalten. Beispielsweise können die erfindungsgemäßen Trägerkatalysatoren Alkalimetall- oder Erdalkalimetallverbindungen, insbesondere Alkalimetall- oder Erdalkalimetalloxide enthalten. Diese können bei der Herstellung der Katalysatoren zugesetzt werden oder in Spuren auch im verwendeten Trägermaterial vorkommen. Zusätzlich können bei der Herstellung des Trägerkatalysators weitere Hilfsstoffe zugegeben werden. Ein Beispiel dafür ist Graphit, welches als Verarbeitungshilfsmittel eingesetzt werden kann.

Die Verfahrensbedingungen zum Betrieb einer Hydrierung in der zweiten Stufe sind dem Fachmann in aller Regel geläufig. Die Hydrierung in der zweiten Hydrierstufe kann im Allgemeinen bei einem Druck von 5 bis 250 bar, vorzugsweise 10 bis 150 bar besonders bevorzugt 15 bis 30 bar durchgeführt werden. Der Druck in der zweiten Hydrierstufe könnte grundsätzlich unabhängig von der ersten Hydrierstufe eingestellt werden. Sofern der Druck in der zweiten Hydrierstufe höher sein soll, ist dafür jedoch ein gewisser apparativer Aufwand notwendig. Es ist deshalb vorteilhaft, wenn der Druck in der zweiten Hydrierstufe geringer ist als in der ersten Hydrierstufe. Die Temperatur bei der Hydrierung in der zweiten Hydrierstufe des erfindungsgemäßen Verfahrens liegt vorzugsweise im Bereich von 100 bis 220 °C, vorzugsweise von 120 °C bis 210 °C, besonders bevorzugt von 140 bis 200 °C.

Die Hydrierung in der zweiten Hydrierstufe kann weiterhin in Gegenwart eines unter den Hydrierbedingungen inerten oder zumindest überwiegend inerten Lösungsmittels durchgeführt werden. Inerte Lösungsmittel sind dem Fachmann bekannt, werden aber vorzugsweise aus der Gruppe, bestehend aus Kohlenwasserstoffen und Alkoholen, vorzugsweise den aus den eingesetzten Aldehyden erhaltenen Alkoholen ausgewählt. Alkohole können zwar an den Trägermaterialien in sehr geringem Umfang zu Ethern umgesetzt werden. Dies ist im Rahmen der vorliegenden Erfindung jedoch dennoch als inert zu verstehen. Darüber hinaus kann die Hydrierung in Anwesenheit einer wässrigen Phase, beispielsweise Prozesswasser aus der Herstellung der Aldehyde, das aus der ersten Hydrierstufe mitgeschleppt wird, oder der Hydrierung selbst, durchgeführt werden. Für die zweite Hydrierstufe kann es weiterhin bevorzugt sein, wenn zu der Hydrierung zusätzlich eine wässrige Phase hinzugefügt wird. Es ist im Rahmen der vorliegenden Erfindung also besonders bevorzugt, dass in der ersten Hydrierstufe keine wässrige Phase hinzugefügt wird, wohingegen in der zweiten Hydrierstufe eine wässrige Phase, beispielsweise Prozesswasser aus der vorherigen Herstellung der Aldehyde oder der Hydrierung selbst, hinzugefügt wird.

Das für die Hydrierung in der zweiten Hydrierstufe eingesetzte Wasserstoff-haltige Gas kann sowohl Wasserstoff sein als auch ein Gasgemisch, welches neben Wasserstoff ein oder mehrere unter den Hydrierbedingungen inerte Gase enthält. Klar sollte sein, dass die Menge an Wasserstoff hoch genug ist, um die Hydrierung in ausreichendem Maße durchführen zu können. Es ist weiterhin bevorzugt, wenn der Wasserstoff im Hinblick auf die zu hydrierenden Aldehyde in einem gewissen stöchiometrischen Überschuss eingesetzt wird. Der stöchiometrische Überschuss an Wasserstoff im Vergleich zu den zu hydrierenden Aldehyden liegt auch in der zweiten Hydrierstufe vorzugsweise im Bereich von 5 bis 90%, besonders bevorzugt zwischen 20 und 70%. Die Hydrierung wird somit vorzugsweise in beiden Hydrierstufen bezogen auf die zu hydrierenden Aldehyde mit einem stöchiometrischen Überschuss an Wasserstoff durchgeführt.

Das aus der zweiten Hydrierstufe erhaltene Reaktionsprodukt, welches zumindest die gebildeten Alkohole und nicht umgesetzte Aldehyde enthält, kann in bekannter Weise aufgearbeitet werden, beispielsweise mit einer Abtrennung des überschüssigen bzw. nicht umgesetzten Wasserstoffs und/oder Produktabtrennung mittels Destillation, Membrantrennung oder anderen geeigneten Verfahren.

### Beispiel 1 (erfindungsgemäß)

Die Hydrierung erfolgte in einer Hydrierstufe mit einem aktivierter Metallkatalysator auf Basis eines Nickelmetallschaums (Katalysator 1). Die Hydrierung wurde mit Isotridecanal als Aldehyd durchgeführt.

### Herstellung des aktivierten Metallkatalysator auf Basis eines Nickelmetallschaums (Katalysator 1)

Ein kommerziell als Rollenware erhältlicher Nickelschaum mit einer Dicke von 1,9 mm, einer Breite von 300 mm und einer mittleren Porengröße von 580 µm wurde mit einer kommerziell erhältlichen Polyethylenimin-Haftvermittlerlösung besprüht und mit einem Aluminiumpulver (Sauerstoffgehalt: 0,5 Gew.-%) enthaltend 96,5 Gew.-% Aluminiumpartikel mit einer Partikelgröße < 150 µm (d₉₀ ≈ 68 µm) beschichtet und einer mehrstufigen Wärmebehandlung unter Sauerstoffausschluss bei maximal 725 °C unterzogen. Dabei wurden die Massenverhältnisse von eingesetztem Nickelschaum und Aluminiumpulver so gewählt, dass das Verhältnis von Aluminium zur Gesamtmasse der geträgerten Legierung bei 28 ± 2 % lag. Nach Abkühlung erfolgte eine Zerkleinerung des Materials mit einem Laser in quaderförmige Teilchen mit einer Kantenlänge von 4x4x1,9 mm. Das resultierende Schüttgut wurde durch 60-minütige Behandlung in einer 10 Gew.%-igen Natronlauge bei 60 °C aktiviert. Anschließend wurde der Katalysator solange mit VE-Wasser gewaschen bis ein pH-Wert < 10 erreicht war.

### Molybdän-Dotierung

250 g des frisch hergestellten Katalysators wurden bei Raumtemperatur über mehrere Stunden mit einer 55,4 Gew.-%igen Ammoniumheptamolybdatlösung behandelt, bis das in der Lösung enthaltende Molybdän vollständig auf dem aktivierten Nickelschaumkatalysator abgeschieden war. Die Kontrolle der Molybdänabscheidung erfolgte mittels Nachweis von Molybdän in der überstehenden Lösung mit Merckoquant oder Quantofix Teststäbchen. Die Behandlung wurde beendet, als kein Molybdän in der überstehenden Lösung mehr nachweisbar war. Anschließend wurde der Katalysator zweimal mit VE-Wasser gewaschen. Der finale Katalysator enthielt mehr als 87 Gew.-% Nickel, etwa 12 Gew.-% Aluminium und weniger als 1 Gew.-% Molybdän.

### Durchführung der Reaktion

Die Hydrierung von Isotridecanal erfolgte in einem Rohrreaktor im Kreislaufbetrieb. Der Kreislaufrohrreaktor hat einen Innendurchmesser von 40 mm und eine Länge von 250 mm. Der Rohrreaktor wurde im Gleichstrom mit Flüssigphase (Isotridecanal und recycliertes Hydrierprodukt) und Gasphase (Wasserstoff) in Rieselbett-Fahrweise durchströmt. In den Kreislaufreaktor wurde 50 ml Katalysator 1 als Hydrierkatalysator eingesetzt. Die in der Hydrierung eingesetzte Menge an Isotridecanal betrug 830 g (ca. 1 L). Der Kreislaufstrom betrug 45 L/h. Die Wasserstoffregelung (max. 2 L/min) erfolgte über eine konstante Abgasfahrweise mit einem Abgasstrom von 1 L/min. Die Versuche wurden jeweils bei einem Anlagendruck von 25 bar im Kreislaufrohrreaktor durchgeführt. Die Reaktion im Kreislaufrohrreaktor wurde bei Reaktionstemperaturen von 130 °C und 150 °C durchgeführt. Der Austrag aus der Hydriereinheit wurde auf den Umsatz des Isotridecanals mittels Gaschromatographie untersucht. Die zu bestimmten Zeitpunkten gemessenen Umsätze sind in Tabelle 2 gezeigt.

**Tabelle 2: Umsatz über die Zeit für Beispiel 1**

| Ni-Schaum | | |
|---|---|---|
| Zeit / Min | Umsatz / % (130 °C) | Umsatz / % (150 °C) |
| 5 | 23,54 | 28,96 |
| 10 | 34,17 | 43,39 |
| 15 | 48,33 | 56,88 |
| 30 | 69,14 | 84,27 |
| 45 | 84,40 | 95,49 |

### Beispiel 2 (nicht erfindungsgemäß)

Beispiel 2 wurde weitestgehend wie Beispiel 1 durchgeführt. Beispiel 2 unterscheidet sich von Beispiel 1 darin, dass im Kreislaufreaktor ein Trägerkatalysator mit Nickel und Kupfer als katalytisch aktiver Komponente und Aluminiumoxid als Trägermaterial (Katalysator 2) eingesetzt wurde. Die Reaktion im Kreislaufrohrreaktor wurde bei Reaktionstemperaturen von 150 °C und 180 °C durchgeführt. Die zu bestimmten Zeitpunkten gemessenen Umsätze sind in Tabelle 3 gezeigt.

**Tabelle 3: Umsatz über die Zeit für Beispiel 2**

| Specialyst^{©} 103 | | |
|---|---|---|
| Zeit / Min | Umsatz / % (150 °C) | Umsatz / % (180 °C) |
| 5 | 10,02 | 23,08 |
| 10 | 15,44 | 34,35 |
| 15 | 20,42 | 44,59 |
| 30 | 34,78 | 69,41 |
| 45 | 48,38 | 84,93 |

Es zeigt sich sehr deutlich, dass sich durch den Einsatz eines aktivierten Metallkatalysator auf Basis eines Nickelmetallschaums in kürzerer Zeit höhere Umsätze erzielt werden können. Zudem kann bei dem aktivierten Metallkatalysator auf Basis eines Nickelmetallschaums auch mit geringeren Temperaturen im Reaktor gearbeitet werden.

### Beispiel 3

Für eine zweistufige Versuchsanordnung wurden die Daten simuliert. Die notwendigen kinetischen Parameter wurden aus den experimentellen Daten der Beispiele 1 und 2 generiert. Für die Simulation wurde festgelegt, dass die Hydrierung von Isotridecanal in einem Rohrreaktor im Kreislaufbetrieb mit einem angeschlossenen zweiten Rohrreaktor im geraden Durchgang erfolgen soll. Der Kreislaufrohrreaktor hat einen Innendurchmesser von 20,5 mm und eine Länge von 730 mm. Der zweite Reaktor hat einen Innendurchmesser von 20,5 mm und eine Länge von 1000 mm. Die Rohrreaktoren werden im Gleichstrom mit Flüssigphase (Isotridecanal und recycliertes Hydrierprodukt) und Gasphase (Wasserstoff) in Rieselbett-Fahrweise durchströmt. In den Kreislaufreaktor werden 100 mL Katalysator 1 als Hydrierkatalysator eingesetzt. Im zweiten Reaktor wurden 100 mL Katalysator 2 verwendet. Die in der Hydrierung eingesetzte Zufuhrrate an Isotridecanal beträgt zwischen 300 und 600 g/h. Der Kreislaufstrom beträgt 25 L/h. Die Wasserstoffregelung (1,6 L/min - 4 ml/min) erfolgt über eine konstante Abgasfahrweise mit einem Abgasstrom von 1 L/min. Im Kreislaufreaktor liegt ein Anlagendruck von 26 bar und im zweiten Rohrreaktor ein Druck von 22,5 bar an. Die Reaktionstemperatur im Kreislaufrohrreaktor wird zwischen 130 und 150 °C variiert. Im zweiten Rohrreaktor wird eine Temperatur von 180 °C angelegt. Der Umsatz des Isotridecanals nach dem zweiten Reaktor beträgt >99 %. Die Reaktionsbedingungen sind in Tabelle 4 aufgeführt. Die entsprechenden Umsätze nach dem Kreislaufreaktor sind in Tabelle 5 dargestellt.

**Tabelle 4: Übersicht über die Hydrierbedingungen**

| | |
|---|---|
| Temperatur Kreislaufreaktor / °C | 130-150 |
| Druck Kreislaufreaktor / bar | 26 |
| Zufuhrrate Isotridecanal/ g h⁻¹ | 300-600 |
| Kreislaufrate Flüssigphase / L h⁻¹ | 25 |
| Volumen Katalysator im Kreislaufreaktor / mL (Katalysator 1) | 100 |
| Länge Katalysatorschüttung / mm | 320 |
| Abgas / NI min⁻¹ | 1 |
| WHSV / g Isotridecanal*(ml Katalysator * h)⁻¹ | 3-6 |

**Tabelle 5: Umsätze nach Kreislaufreaktor bei verschiedenen Hydrierbedingungen**

| Temperatur/ °C WHSV / g Isotridecanal*(ml Katalysator * h)⁻¹ | 130 | 150 |
|---|---|---|
| 3 | 93,37 % | 97,31 % |
| 6 | 87,07 % | 94,59 % |

### Beispiel 4 (nicht erfindungsgemäß)

Beispiel 4 wurde weitestgehend wie Beispiel 3 durchgeführt. Beispiel 4 unterscheidet sich von Beispiel 3 darin, dass in der ersten Hydrierstufe und in der zweiten Hydrierstufe jeweils Katalysator 2 eingesetzt wird. Zudem wird die Zufuhrrate des Isotridecanals auf 270 - 530 g/h reduziert. Weiterhin werden im Kreislaufreaktor höhere Temperaturen von 150 - 170 °C eingestellt. Der Umsatz des Isotridecanals nach dem zweiten Reaktor beträgt hier ebenfalls >99 %. Eine Übersicht über die Hydrierbedingungengen findet sich in nachfolgender Tabelle 6 und die berechneten Umsätze bei den verschiedenen Reaktionsbedigungen sind in Tabelle 7 aufgeführt.

**Tabelle 6: Übersicht über die Hydrierbedingungen**

| Temperatur Kreislaufreaktor / °C | 150-170 |
|---|---|
| Druck Kreislaufreaktior / bar | 26 |
| Zufuhrrate Isotridecanal / g h⁻¹ | 270-530 |
| Kreislaufrate Flüssigphase / L h⁻¹ | 25 |
| Volumen Katalysator im Kreislaufreaktor / mL (Katalysator 2) | 100 |
| Länge Katalysatorschüttung / mm | 640 |
| Abgas / NI min⁻¹ | 1 |
| WHSV / g Isotridecanal*(ml Katalysator * h)⁻¹ | 2,7-5,3 |

**Tabelle 7: Umsätze nach Kreislaufreaktor bei verschiedenen Hydrierbedingungen**

| Temperatur/ °C WHSV / g Isotridecanal*(ml Katalysator * h)⁻¹ | 150 | 170 |
|---|---|---|
| 2,7 | 93,30 % | 96,31 % |
| 5.3 | 86,99 % | 92,83 % |

Es zeigt sich sehr deutlich, dass sich durch den Einsatz eines aktivierten Metallkatalysator auf Basis eines Nickelmetallschaums in der ersten Hydrierstufe bei gleichbleibenden Umsätzen von > 99% signifikant höhere Zufuhrraten einstellen lassen. Zudem kann bei dem aktivierten Metallkatalysator auf Basis eines Nickelmetallschaums auch mit geringeren Temperaturen im Kreislaufreaktor gearbeitet werden.

## Patentansprüche

1. Verfahren zur Herstellung von Alkoholen durch kontinuierliche Hydrierung von C13-Aldehyden in mindestens zwei Hydrierstufen, wobei
ein Strom, der die zu hydrierenden C13-Aldehyde enthält, in flüssiger Phase in der ersten Hydrierstufe, die mindestens einen Kreislaufreaktor umfasst, mit einem Wasserstoff-haltigen Gas an einem aktivierten Metallkatalysator auf Basis eines Nickelmetallschaums hydriert wird, wobei aus dem mindestens einen Kreislaufreaktor ein Rohproduktstrom, zumindest enthaltend Alkohole und nicht umgesetzte Aldehyde, entnommen wird, von dem ein erster Teil zurückgeführt und ein zweiter Teil zur zweiten Hydrierstufe geführt wird,
der zweite Teil des Rohproduktstroms in flüssiger Phase in der zweiten Hydrierstufe, die mindestens einen im geraden Durchgang betriebenen Reaktor umfasst, mit einem Wasserstoff-haltigen Gas an einem Trägerkatalysator hydriert wird, der eine katalytisch aktive Komponente und ein Trägermaterial umfasst, wobei die katalytisch aktive Komponente aus der Gruppe bestehend aus Nickel, Kupfer, Chrom und Mischungen davon ausgewählt wird, und wobei das Trägermaterial zu mehr als 90 Gew.-% aus einem oxidischen Material besteht, welches aus der Gruppe bestehend aus Aluminiumoxid, Aluminiumsilikat, Siliciumdioxid, Titandioxid, Zirkonoxid und Mischungen von zwei oder mehr davon, ausgewählt wird.

2. Verfahren nach Anspruch 1, wobei der beim Verfahren eingesetzte Strom ein Isotridecanal-Strom ist.

3. Verfahren nach Anspruch 1 oder 2, wobei der aktivierte Metallkatalysator auf Basis eines Nickelmetallschaums frei von organischen Bestandteilen ist, d. h. die Summe der Gewichtsanteile von Kohlenstoff und kohlenstoffhaltigen Verbindungen weniger als 0,2 Gew.-% des Gesamtgewichts des Katalysators beträgt.

4. Verfahren nach einem der vorhergehenden Ansprüche, wobei der aktivierte Metallkatalysator auf Basis eines Nickelmetallschaums 80 bis 95 Gew.-% Nickel und 5 bis 15 Gew.-% Aluminium, jeweils bezogen auf das Gesamtgewicht des Katalysators, enthält.

5. Verfahren nach Anspruch 4, wobei der aktivierte Metallkatalysator auf Basis eines Nickelmetallschaums zusätzlich 0,01 bis 3 Gew.-% Molybdän, besonders bevorzugt 0,2 bis 1,5 Gew.-% Molybdän und ganz besonders bevorzugt 0,3 bis 0,7 Gew.-%, jeweils bezogen auf das Gesamtgewicht des Katalysators, enthält.

6. Verfahren nach einem der vorhergehenden Ansprüche, wobei die Hydrierung in der ersten Hydrierstufe bei einem Druck von 5 bis 150 bar, vorzugsweise 15 bis 50 bar, besonders bevorzugt 20 bis 45 bar durchgeführt wird.

7. Verfahren nach einem der vorhergehenden Ansprüche, wobei die Hydrierung in der ersten Hydrierstufe bei einer Temperatur von 50 bis 250 °C, vorzugsweise 80 bis 200 °C, besonders bevorzugt von 100 bis 190 °C durchgeführt wird.

8. Verfahren nach einem der vorhergehenden Ansprüche, wobei der Umsatz der Hydrierung in der ersten Hydrierstufe mindestens 85%, vorzugsweise mindestens 90%, besonders bevorzugt mindestens 95% beträgt.

9. Verfahren nach einem der vorhergehenden Ansprüche, wobei der Volumenanteil des aktivierten Metallkatalysators auf Basis eines Nickelmetallschaums am Gesamtkatalysatorvolumen aller Hydrierstufen von 30 bis 80%, vorzugsweise 35 bis 60% beträgt.

10. Verfahren nach einem der vorhergehenden Ansprüche, wobei das Trägermaterial des Trägerkatalysators Aluminiumoxid, Aluminiumsilikat oder Siliciumdioxid ist.

11. Verfahren nach Anspruch 10, wobei das Trägermaterial eine BET-Oberfläche von 70 bis 350 m²/g, vorzugsweise 150 bis 280 m²/g aufweist.

12. Verfahren nach einem der vorhergehenden Ansprüche, wobei die Hydrierung in der zweiten Hydrierstufe bei einem Druck von 5 bis 250 bar, vorzugsweise 10 bis 150 bar besonders bevorzugt 15 bis 30 bar durchgeführt wird.

13. Verfahren nach einem der vorhergehenden Ansprüche, wobei die Hydrierung in der zweiten Hydrierstufe bei einer Temperatur von 100 bis 220 °C, vorzugsweise 120 °C bis 210 °C, besonders bevorzugt 140 bis 200 °C durchgeführt wird.

14. Verfahren nach einem der vorhergehenden Ansprüche, wobei die Hydrierung in beiden Hydrierstufen bezogen auf die zu hydrierenden Aldehyde mit einem stöchiometrischen Überschuss an Wasserstoff erfolgt.

15. Verfahren nach einem der vorhergehenden Ansprüche, wobei in der ersten Hydrierstufe keine wässrige Phase, in der zweiten Hydrierstufe hingegen eine wässrige Phase hinzugefügt wird.
